# EUROPEAN PATENT APPLICATION

(11) **EP 2 127 653 A1**
(43) Date of publication of application: **02.12.2009**
(21) Application number: 08710303.2
(22) Date of filing: 28.01.2008
(51) Int. Cl.: A61K 31/50, A61P 25/00, C07D 237/14

(54) **AGENT FOR TREATMENT OF MULTIPLE SCLEROSIS**

(30) Priority: 29.01.2007 JP 2007017328
(71) Applicant: Kowa Co., Ltd., Nagoya-shi, Aichi 460-8625 (JP)
(72) Inventor: YAMABI, Masaki, Higashimurayama-shi Tokyo 189-0022 (JP); TABUNOKI, Yuichiro, Higashimurayama-shi Tokyo 189-0022 (JP); EDANO, Toshiyuki, Higashimurayama-shi Tokyo 189-0022 (JP)
(74) Representative: Hartz, Nikolai
(86) International application number: PCT/JP2008/000099
(87) International publication number: WO 2008/093495

(57) **Abstract**

To provide a preventive and/or therapeutic drug for multiple sclerosis, which has high efficacy against multiple sclerosis, which exhibits excellent safety, and which can be orally administered.

The invention provides a preventive and/or therapeutic drug for multiple sclerosis, the drug containing, as an active ingredient, 2-benzyl-5-(4-chlorophenyl)-6-[4-(methylthio)phenyl]-2H-pyridazin-3-one or a solvate thereof.

## Description

### [Technical Field]

The present invention relates to a novel preventive and/or therapeutic drug for multiple sclerosis.

### [Background Art]

Multiple sclerosis is a cryptogenic, intractable disease which is designated as a disease specified by the Japanese Ministry of Health, Labor and Welfare, and this disease involves central dysfunction due to cerebrospinal demyelination and causes sight problems, dyskinesia, etc. In Europe and the United States, the prevalence of multiple sclerosis is higher than that of any other neurological diseases affecting young adults, and is about 50 per 100,000 population. In Japan, the prevalence of multiple sclerosis is estimated to be about 8 or 9 per 100,000 population, and the number of multiple sclerosis patients is estimated to be about 12,000. In many cases, multiple sclerosis involves repeated remissions and relapses (relapsing-remitting multiple sclerosis). Particularly, the onset of multiple sclerosis often occurs around age 30, and relapse of the disease becomes less frequent with increasing age. However, some multiple sclerosis patients exhibit rapid progression of symptoms (chronic progressive multiple sclerosis). As has been shown, T-cells or macrophages infiltrate into lesion sites of multiple sclerosis patients, and these cells are considered to break myelin protein and myelin sheath in central nerves, thereby causing neurological disorders (Non-Patent Document 1). Demyelinating lesions may be distributed throughout the central nervous system, but are located mainly in, for example, the optic nerves, the brain stem, the spinal cord, and the cerebellum. Multiple sclerosis causes a variety of symptoms (e.g., paralysis of the limbs, shivering, fatigue, optic nerve disorder, dysuria, and dyschezia), and these symptoms vary depending on the site where nerves are damaged. Although the mechanism and other features of multiple sclerosis onset have not been elucidated in detail, multiple sclerosis is considered a type of autoimmune disease, since an immunosuppressive agent effectively acts on the disease (Non-Patent Document 2). Meanwhile, some studies suggest that activation of the immune system associated with viral infection or bacterial infection contributes to the onset of multiple sclerosis (Non-Patent Document 3).
In practice, characteristic infiltration of T-cells or monocytes/macrophages was found in lesion sites of multiple sclerosis patients, and it has been suggested that Th1 cytokines (e.g., interferon-γ) produced by such cells are associated with multiple sclerosis symptoms (Non-Patent Document 4). Therefore, conceivably, suppression of infiltration of such cells into the lesion sites could provide a promising therapeutic effect on multiple sclerosis.

In current therapy for multiple sclerosis, a steroid is used in the onset phase, whereas an interferon-β (IFN-β) pharmaceutical product or a glatiramer pharmaceutical product is used in the remission phase. An IFN-β pharmaceutical product acts on T-cells, to thereby control the amount of interferon-γ produced, and to suppress antigen presentation by antigen-presenting cells (Non-Patent Document 5). Therefore, conceivably, an IFN-β pharmaceutical product suppresses enhancement of immune response, and contributes to delay of progression of multiple sclerosis symptoms or reduction in relapse frequency. However, even when such a pharmaceutical product is administered to multiple sclerosis patients, relapses of multiple sclerosis occur (average relapse frequency: about 0.8 a year), and a method for reliably preventing relapse of multiple sclerosis has not yet been developed (Non-Patent Document 6). In addition, such a pharmaceutical product requires frequent subcutaneous administration, which causes problematic side effects such as fever and subcutaneous ulcer, and imposes a considerable burden on patients in terms of compliance. Therefore, development of a pharmaceutical product which can be orally administered is considered very important for the treatment of multiple sclerosis. Meanwhile, although pulse therapy using cyclophosphamide (i.e., an immunosuppressive agent) has been reported to be effective for patients with severe progressive multiple sclerosis, this agent causes severe side effects (e.g., leukopenia and alopecia), and thus requires careful administration. Under such circumstances, keen demand has arisen for an effective therapeutic method for multiple sclerosis.

Patent Document 1 discloses that 2-benzyl-5-(4-chlorophenyl)-6-[4-(methylthio)phenyl]-2H-pyridazin-3-one, which is a known substance, exhibits an excellent interleukin-1β production inhibitory effect, and this substance is applied to a preventive or therapeutic drug for, for example, rheumatism, arthritis, osteoporosis, inflammatory colitis, immunodeficiency syndrome, sepsis, hepatitis, nephritis, ischemic disease, insulin-dependent diabetes, arteriosclerosis, Parkinson's disease, Alzheimer's disease, or leukemia. However, application of this substance to multiple sclerosis has not yet been reported.
[Patent Document 1] WO 99/25697 pamphlet
[Non-Patent Document 1] Noseworthy, et al.; N. Engl. J. Med., Vol. 343, No. 13, pp. 938-952 (2000)
[Non-Patent Document 2] Neuhaus, et al.; Trends Pharmacol. Sci., Vol. 24, No. 3, pp. 131-138 (2003)
[Non-Patent Document 3] Tejada-Simon, et al.; Ann. Neurol., Vol. 53, pp. 189-97 (2003)
[Non-Patent Document 4] Bielekova, et al.; Nat. Med., Vol. 6, pp. 1167-75 (2000)
[Non-Patent Document 5] Joseph, et al.; J. Neuroimmunol., Vol. 20, No. 1, pp. 39-44 (1988)
[Non-Patent Document 6] The IFNB Multiple Sclerosis Study Group; Neurology, Vol. 43, No. 4, pp. 655-61 (1993)

### [Disclosure of the Invention]

### [Problems to be Solved by the Invention]

An object of the present invention is to provide a preventive and/or therapeutic drug for multiple sclerosis, which has high efficacy against multiple sclerosis, which exhibits excellent safety, and which can be orally administered.

### [Means for Solving the Problems]

In view of the foregoing, the present inventors have conducted extensive studies, and as a result have found that administration of 2-benzyl-5-(4-chlorophenyl)-6-[4-(methylthio)phenyl]-2H-pyridazin-3-one exhibits an excellent effect of suppressing symptoms of multiple sclerosis. The present invention has been accomplished on the basis of this finding.

Accordingly, the present invention provides:
1) a preventive and/or therapeutic drug for multiple sclerosis, the drug containing, as an active ingredient, 2-benzyl-5-(4-chlorophenyl)-6-[4-(methylthio)phenyl]-2H-pyridazin-3-one or a solvate thereof;
2) a pharmaceutical composition for prevention and/or treatment of multiple sclerosis, the composition comprising 2-benzyl-5-(4-chlorophenyl)-6-[4-(methylthio)phenyl]-2H-pyridazin-3-one or a solvate thereof, and a pharmaceutically acceptable carrier;
3) a method for prevention and/or treatment of multiple sclerosis, the method comprising administering, in an effective amount, 2-benzyl-5-(4-chlorophenyl)-6-[4-(methylthio)phenyl]-2H-pyridazin-3-one or a solvate thereof to a subject in need thereof;
4) use of 2-benzyl-5-(4-chlorophenyl)-6-[4-(methylthio)phenyl]-2H-pyridazin-3-one or a solvate thereof for producing a preventive and/or therapeutic drug for multiple sclerosis; and
5) 2-benzyl-5-(4-chlorophenyl)-6-[4-(methylthio)phenyl]-2H-pyridazin-3-one or a solvate thereof for prevention and/or treatment of multiple sclerosis.

### [Effects of the Invention]

The preventive and/or.therapeutic drug for multiple sclerosis of the present invention can be orally administered, and exhibits an excellent effect of suppressing multiple sclerosis. Therefore, the drug is effective for prevention and/or treatment of multiple sclerosis.

### [Brief Description of the Drawings]

[Fig. 1] Fig. 1 is a graph showing change in scores, which correspond to the effects of drugs on experimental autoimmune encephalomyelitis symptoms.
[Fig. 2] Fig. 2 is a graph showing maximum scores, which correspond to the effects of drugs on experimental autoimmune encephalomyelitis symptoms.

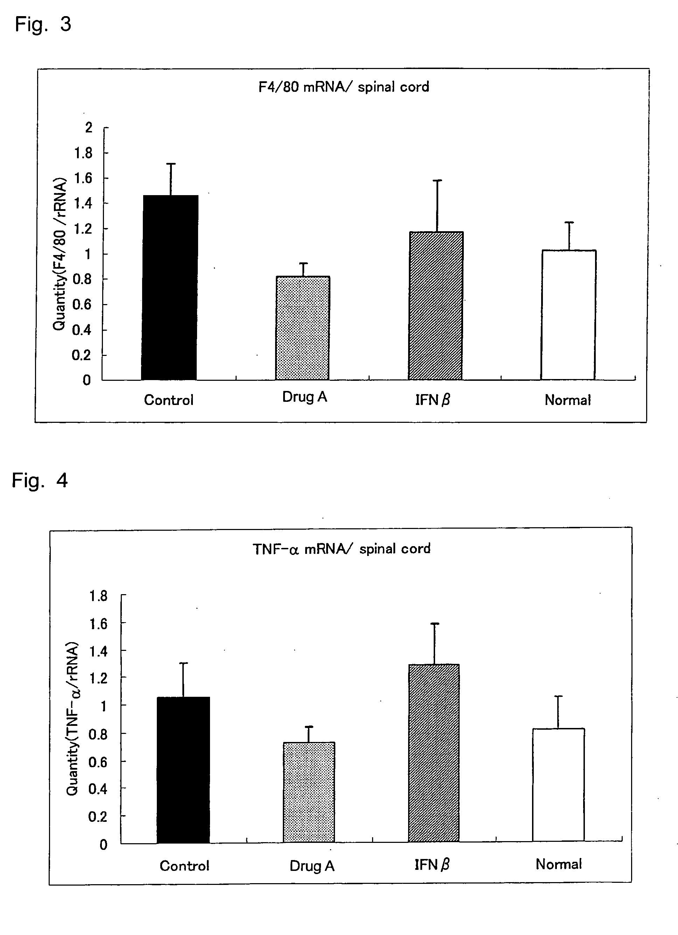

[Fig. 3] Fig. 3 is a graph showing the effects of drugs on the amount of F4/80 mRNA expression in the spinal cord of experimental autoimmune encephalomyelitis mice.
[Fig. 4] Fig. 4 is a graph showing the effects of drugs on the amount of TNF-α mRNA expression in the spinal cord of experimental autoimmune encephalomyelitis mice.

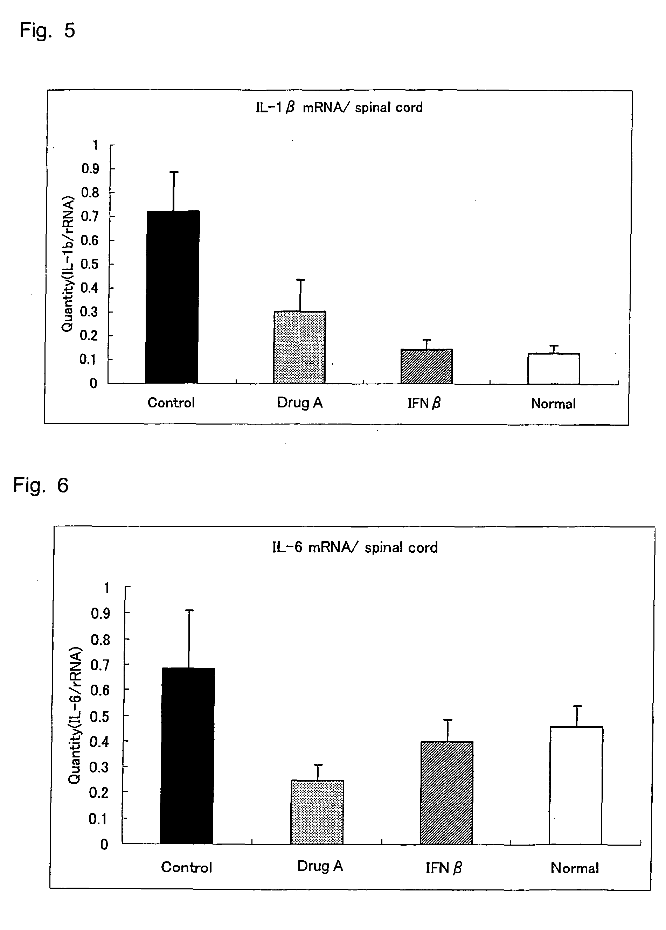

[Fig. 5] Fig. 5 is a graph showing the effects of drugs on the amount of IL-1β mRNA expression in the spinal cord of experimental autoimmune encephalomyelitis mice.
[Fig. 6] Fig. 6 is a graph showing the effects of drugs on the amount of IL-6 mRNA expression in the spinal cord of experimental autoimmune encephalomyelitis mice.

### [Best Modes for Carrying Out the Invention]

The drug of the present invention for the prevention and/or treatment of multiple sclerosis employs 2-benzyl-5-(4-chlorophenyl)-6-[4-(methylthio)phenyl]-2H-pyridazin-3-one, which is a known substance. This compound can be produced through, for example, a method described in WO 99/25697 or a similar method.

2-Benzyl-5-(4-chlorophenyl)-6-[4-(methylthio)phenyl]-2H-pyridazin-3-one may be in the form of a solvate (typically a hydrate). The present invention encompasses such a solvate.

As described in the Examples hereinbelow, in an evaluation system employing an experimental autoimmune encephalomyelitis mouse model, which is a widely known animal model of multiple sclerosis, administration of 2-benzyl-5-(4-chlorophenyl)-6-[4-(methylthio)phenyl]-2H-pyridazin-3-one or a solvate thereof was suggested to exhibit the effect of suppressing infiltration of macrophages into the spinal cord; i.e., an excellent effect of suppressing multiple sclerosis. Therefore, the present invention realizes prevention of the onset of multiple sclerosis, prevention of relapse thereof, suppression of progression thereof, treatment thereof, and amelioration of pathological conditions thereof.

No particular limitation is imposed on the dosage form of the preventive and/or therapeutic drug for multiple sclerosis of the present invention, and the dosage form may be appropriately determined in consideration of the purpose of treatment. For example, the preventive and/or therapeutic drug may be orally administered in the form of, for example, tablet, capsule, granule, film coating agent, powder, or syrup, or may be parenterally administered in the form of, for example, injection, suppository, inhalant, percutaneous absorption agent, eye drop, or nasal drop. The preventive and/or therapeutic drug is preferably provided in the form of a peroral product.

A pharmaceutical product suitable for such a dosage form may contain a pharmaceutically acceptable carrier. Examples of the carrier include excipients and extenders such as starches, lactose, sucrose, mannitol, and silicic acid; disintegrating agents such as agar, calcium carbonate, potato or tapioca starch, alginic acid, and composite silicic acid salts; binders such as hydroxypropylmethylcellulose, methylcellulose, sodium carboxymethylcellulose, alginic acid salts, gelatin, polyvinyl pyrrolidone, sucrose, and gum arabic; lubricants such as talc, calcium stearate, magnesium stearate, solid polyethylene glycols, sodium laurylsulfate, and mixtures thereof; diluents such as lactose and cornstarch; buffers such as organic acids (e.g., citric acid, phosphoric acid, tartaric acid, and lactic acid), inorganic acids (e.g., hydrochloric acid), alkali hydroxides (e.g., sodium hydroxide and potassium hydroxide), and organic amines (e.g., triethanolamine, diethanolamine, and diisopropanolamine); antiseptics such as p-hydroxybenzoic acid esters and benzalkonium chloride; emulsifiers such as anionic surfactants (e.g., calcium stearate, magnesium stearate, and sodium laurylsulfate), cationic surfactants (e.g., benzalkonium chloride, benzethonium chloride, and cetylpyridinium chloride), and nonionic surfactants (e.g., glyceryl monostearate, sucrose fatty acid esters, polyoxyethylene hydrogenated castor oil, polyoxyethylene sorbitan fatty acid esters, polyoxyethylene fatty acid esters, and polyoxyethylene alkyl ethers); and stabilizers such as sodium sulfite, sodium hydrogensulfite, dibutylhydroxytoluene, butylhydroxyanisole, and edetic acid. Optionally, the pharmaceutical product may appropriately contain an additional additive (e.g., a flavoring agent, a dispersant, a preservative, or a perfume) in combination with any of the aforementioned carriers.

In the present invention, the dose of 2-benzyl-5-(4-chlorophenyl)-6-[4-(methylthio)phenyl]-2H-pyridazin-3-one or a solvate thereof may be appropriately determined in consideration of the body weight, age, sex, symptom, etc. of a patient in need thereof. Generally, the daily dose of this substance or a solvate thereof for an adult is 2 to 320 mg, preferably 4 to 160 mg. The daily dose is administered once a day, or in a divided manner (several times a day).

### [Examples]

The present invention will next be described in more detail by way of examples. However, the technical scope of the present invention is not limited to these examples.

### Example 1

2-Benzyl-5-(4-chlorophenyl)-6-[4-(methylthio)phenyl]-2H-pyridazin-3-one (hereinafter will be referred to as "drug A") was administered to mice of an experimental autoimmune encephalomyelitis model, and symptoms of the disease were observed, whereby the drug was evaluated. The experimental autoimmune encephalomyelitis model is established by immunizing a mouse with myelin protein (i.e., myelin-sheath-constituting protein), thereby causing T-cell-mediated autoimmune inflammation in the central nervous system. This model, in which T-cells or monocytes/macrophages infiltration into the central nervous system, is known as a model exhibiting behaviors similar to pathological conditions of multiple sclerosis.

### A. Material and method

Seven-week-old female C57BL/6 mice (Charles River Laboratories Japan, Inc.) were employed. A sensitizing emulsion for causing the mice to develop experimental autoimmune encephalomyelitis was prepared by mixing myelin oligodendrocyte glycoprotein (MOG) 35-55 (Alexis Biochemical) with an equiamount of complete Freund's adjuvant H37Ra (DIFCO) so that the MOG concentration was 2 mg/mL, followed by homogenization under ice cooling by means of Handy Micro Homogenizer (product of Microtec Co., Ltd.). The thus-prepared emulsion (0.1 mL) was intradermally administered to each mouse at its tail base for initial sensitization. On the same day, pertussis toxin dissolved in saline (500 ng/0.3 mL) was intravenously injected to the tail. Two days after initial sensitization, in a manner similar to that described above, pertussis toxin (500 ng/0.3 mL) was intravenously injected for booster. Drug A was orally administered (30 mg/kg) twice a day in the morning (9:00 to 11:00) and in the evening (15:30 to 17:30) from the day following the initial sensitization to 42 days after sensitization. IFN-β (Nihon Schering) was subcutaneously administered every other day at a dose of 10,000 IU/individual.
The drugs were evaluated from the condition of experimentally created autoimmune encephalomyelitis according to the following ratings:
score 0: no symptom;
score 1: limp tail or abnormal gait;
score 2: limp tail and abnormal gait;
score 3: paralysis of one limb;
score 4: paralysis of two or more limbs; and
score 5: death.

### B. Results

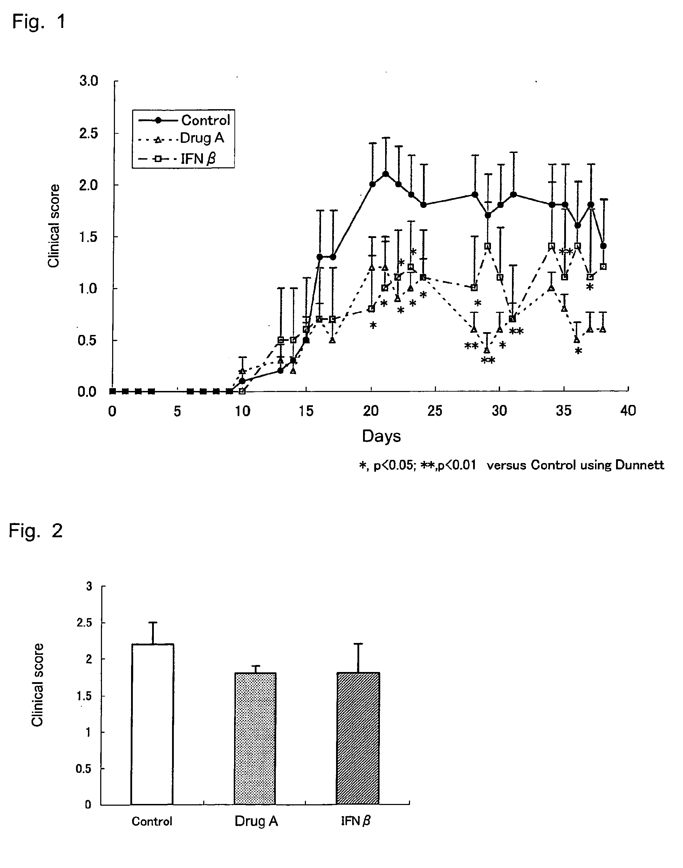

Fig. 1 shows change in scores for drug evaluation; Table 1 shows the results of analysis of the time required for the onset of the disease in individual mice of the tested groups; and Fig. 2 shows the results of analysis of maximum scores of individual mice of the tested groups. In Figs. 1 and 2 and Table 1, the data for each group (10 mice) are represented by average value ± standard error (non-parametric Dunnett's test).
During the experiment, a death case was observed in a control group 16 days after sensitization, and also in an IFN-β administration group 12, 37, and 40 days after sensitization. In contrast, no death case was observed in a drug A administration group. Through oral administration of drug A, experimental autoimmune encephalomyelitis was ameliorated to almost the same extent as in the case of IFN-β (see scores in Fig. 1); the onset of symptoms corresponding to score 2 or worse tended to be delayed (Table 1); and the maximum score of experimental autoimmune encephalomyelitis symptoms tended to be reduced in each mouse (Fig. 2).

**[Table 1]**

| Results of analysis of the number of days required for the onset of experimental autoimmune encephalomyelitis | | |
|---|---|---|
| | Symptoms corresponding to score 2 or worse | |
| | Incidence rate | Days required for onset (days) |
| Control | 9/10 | 18.3 ± 0.8 |
| Drug A | 8/10 | 22.4 ± 1.5 |
| IFN-β | 5/10 | 27.4 ± 3.6* |

| | | |
|---|---|---|
| *: p < 0.05 versus Control using Dunnett | | |

### Example 2

### A. Material and method

On day 42, the spinal cord was removed from the mice employed in Example 1, and total RNA was recovered by use of ISOGEN (Nippon Gene Co., Ltd.). For comparison, similarly, the spinal cord was extirpated from unsensitized normal mice, and was employed for the below-described quantitative determination. cDNA was synthesized from the thus-obtained total RNA by use of a reverse transcriptase, and the amount of mRNA of the following gene (F4/80, TNF-α, IL-1β, or IL-6) was quantitatively determined by means of an ABI PRISM 7900HT system (Applied Biosystems) through real-time PCR employing a TaqMan probe according to the protocol of Applied Biosystems. Meanwhile, the amount of ribosomal RNA was determined, and employed for correction.

### B. Results

As compared with normal mice, the experimental autoimmune encephalomyelitis model mice showed higher amounts of mRNA for the above respective genes. In contrast, in the mice of the drug A administration group, the amount of expression of mRNA of F4/80, which is expressed specifically in monocytes/macrophages, was found to be reduced. This suggests that drug A exhibits the effect of suppressing infiltration of monocytes/macrophages into the spinal cord. Also, in the mice of the drug A administration group, expression of the cytokines TNF-α, IL-1β, and IL-6, which are generally produced by these cells and are suggested to be closely related to pathological conditions of multiple sclerosis, was found to be reduced. Meanwhile, in the mice of the IFN-β administration group, expression of IL-1β and IL-6 was reduced, but expression of TNF-α was not reduced. IFN-β exhibited an F4/80 mRNA expression suppressing effect lower than that of drug A.

As is clear from the aforementioned data, drug A of the present invention or a solvate thereof is effective for, for example, prevention of the onset of multiple sclerosis, prevention of relapse thereof, suppression of progression thereof, treatment thereof, or amelioration of pathological conditions thereof, and thus is useful as a preventive and/or therapeutic drug for multiple sclerosis.

## Claims

1. A preventive and/or therapeutic drug for multiple sclerosis containing, as an active ingredient, 2-benzyl-5-(4-chlorophenyl)-6-[4-(methylthio)phenyl]-2H-pyridazin-3-one or a solvate thereof.

2. The preventive and/or therapeutic drug for multiple sclerosis as described in claim 1, which is a peroral product.

3. Use of 2-benzyl-5-(4-chlorophenyl)-6-[4-(methylthio)phenyl]-2H-pyridazin-3-one or a solvate thereof for producing a preventive and/or therapeutic drug for multiple sclerosis.

4. Use as described in claim 3, wherein the preventive and/or therapeutic drug is a peroral drug.

5. A method for preventing and/or treating for multiple sclerosis, comprising administering an effective amount of 2-benzyl-5-(4-chlorophenyl)-6-[4-(methylthio)phenyl]-2H-pyridazin-3-one or a solvate thereof to a patient in need thereof.

6. The method as described in claim 5, wherein the mode of administration is peroral.
